(19) **Europäisches Patentamt**
**European Patent Office**
**Office européen des brevets**

(11) **EP 1 355 696 B1**

(12) **EUROPEAN PATENT SPECIFICATION**

(45) Date of publication and mention
of the grant of the patent:
**13.05.2009 Bulletin 2009/20**

(51) Int Cl.:
***A61N 7/02*** (2006.01)

(21) Application number: **02709235.2**

(86) International application number:
**PCT/US2002/002724**

(22) Date of filing: **30.01.2002**

(87) International publication number:
**WO 2002/060525 (08.08.2002 Gazette 2002/32)**

(54) **ULTRASOUND WOUND TREATMENT DEVICE USING STANDING WAVES**

VORRICHTUNG ZUR ULTRASCHALL-WUNDBEHANDLUNG UNTER VERWENDUNG
STEHENDER WELLEN

DISPOSITIF POUR TRAITEMENT DE PLAIES PAR ULTRASONS  UTILISANT DES ONDES
STATIONNAIRES

(84) Designated Contracting States:
**AT BE CH CY DE DK ES FI FR GB GR IE IT LI LU
MC NL PT SE TR**

(30) Priority: **30.01.2001   US 774145**

(43) Date of publication of application:
**29.10.2003   Bulletin 2003/44**

(73) Proprietor: **Celleration, Inc.
Eden Prairie, MN 55344 (US)**

(72) Inventor: **BABAEV, Eilaz
Minnetonka, MN 55343 (US)**

(74) Representative: **HOFFMANN EITLE
Patent- und Rechtsanwälte
Arabellastrasse 4
81925 München (DE)**

(56) References cited:
**EP-A- 0 619 104          US-A- 5 152 289
US-A- 5 664 570          US-A- 5 895 362
US-A- 6 007 499          US-A- 6 036 661**

## Description

### FIELD OF INVENTION

The present invention relates to a system for treating a wound, specifically, for the treatment of a wound using ultrasound standing waves. Parts of the following disclosure relate to a method and device for creating ultrasonic standing waves in air and directing them to a wound for delivering aerodynamic forces as ultrasonic radiation pressure and ultrasonic waves as well. Ultrasound radiation pressure so created may be used in treatment to increase the blood flow in a wound area, and to kill bacteria, stimulate healthy tissue cells, and treat the wound with ultrasound waves.

### BACKGROUND OF THE INVENTION

Ultrasonic waves have been widely used in medical applications, including diagnostics and therapy as well as many industrial applications, e. g., welding, cutting, fiber optics technology, speed meters, etc. Diagnostic use of ultrasound waves includes using ultrasonic waves to detect underlying structures in an object or human tissue. In this method, an ultrasonic transducer is placed in contact with the tissue or object via a coupling medium, and high frequency (1-10 MHz) ultrasonic waves are directed into the tissue. Upon contact with the various underlying structures, the waves are reflected back to a receiver adjacent the transducer. By comparison of the signals of the ultrasonic waves sent with the reflected ultrasonic wave as received, an image of the underlying structure can be produced. This technique is particularly useful for identifying boundaries between components of tissue and can be used to detect irregular masses, tumors, etc.

Three therapeutic medical uses of ultrasound waves include aerosol mist production, contact physiotherapy, and soft tissue ablation. The ultrasound contact physiotherapy procedure may cause a patient significant discomfort and/or pain, and skin may appear raw and damaged. Aerosol mist production makes use of a nebulizer or inhaler to produce an aerosol mist for creating a humid environment and delivering drugs to the lungs.

Ultrasonic nebulizers operate by passing ultrasound waves of sufficient intensity through a liquid, the waves being directed at an air-liquid interface of the liquid from a point underneath or within the liquid. Liquid particles are ejected from the surface of the liquid into the surrounding air following the disintegration of capillary waves produced by the ultrasound. This technique can produce a very fine dense fog or mist.

Aerosol mists produced by ultrasound are preferred because a smaller particle size of the aerosol can be obtained with the ultrasonic waves. One of the major shortcomings of ultrasonic inhalers and nebulizers is that there is no directed aerosol to the target. An air stream is then required to direct the aerosol to the target, but this decreases the efficiency of ultrasound.

Ultrasonic sprayers (Sonic and Materials Inc., Misonix Inc., Sono-Tek Inc., Zevex International, Inc.), operate by passing liquid through a central orifice of an ultrasound instrument-tip. See, for example, U.S. Patents Nos. 3,765,606; 4,659,014; 5,104,042; 4,930,700; 4,153,201; 4,655,393; 5,516,043; 5,835,678; 5,879,364; and 5,843,139.

Ultrasonic inhalers and drug delivery systems from Medisonic USA, Inc., 3M, Siemens Gmb, The Procter & Gamble Company, Sheffield Pharmaceuticals, Aradigm, Inc., operate by atomizing liquid using piezoceramic film. See, for example, U.S. Patents Nos. 4,294,407; 5,347,998; 5,520,166; 5,960,792; 6,095,141; 6,102,298; 6,098,620; 6,026,808; and 6,106,547.

US 6,036,661 A discloses an apparatus for administering acoustic shock waves from a pressure pulse source 3a through a fluid medium 3b via a focussing device 3c within the closed structural unit of the treatment head 3. Head 3 is placed against the body of the patient to be treated to direct acoustic energy to an internal treatment location. This device is of the contact physiotherapy type discussed above.

An apparatus for treating a target region with ultrasound standing waves is disclosed in US-A-5,664,570.

### SUMMARY OF THE INVENTION

According to the present invention, there is provided a system for treating a wound with ultrasound standing waves, comprising a generator for generating ultrasound waves, an ultrasound transducer operatively connected to said generator and having a distal radiation surface, and means for adjusting the distance d between the distal radiation surface and a surface of a wound, when the distal radiation surface is active and directed to the surface of the wound, so as to create ultrasound standing waves through air from the distal radiation surface to the surface of the wound when the distance d is determined by the formular $d = n \times \lambda/2$, wherein $\lambda$ is the wavelength of the ultrasound standing wave and n is a positive integer.

Embodiments of the present invention are able to provide an improved method and device for treating wounds.

It is also possible with embodiments of this invention to provide an improved method and device for treating wounds using ultrasound standing waves.

It is further possible with embodiments of the invention to provide a method and device for increasing blood flow, killing bacteria, and stimulating healthy tissue cell growth.

These and other advantages achievable with embodiments of the invention will become more apparent from the discussion below.

With the use of embodiments of the invention, ultrasound waves can be created, directed, and delivered to a wound surface through the air to increase blood flow, kill bacteria, stimulate healthy tissue cells and treat wounds with ultrasound energy. A method of treating wounds with systems of the invention may involve the use of ultrasound standing waves of a continuous or pulsed ultrasound.

Using systems of the invention to treat a wound may comprise producing ultrasound standing waves using a free end surface of an ultrasonic transducer and a wound surface.

In one treatment application using systems of the present invention, the radiation pressure of ultrasonic standing waves can increase blood flow in the wound area and destroy surface bacteria to result in a higher disinfecting property of ultrasound. Additionally, the ultrasound can also stimulate healthy cell growth to aid in granulization and epithelization of the healing tissue. Other contemplated applications of the system include non-medical uses such as cleansing, drying, sterilizing and coating surfaces of objects and food:

Systems of the present invention can offer an approach that may re-establish use of some traditional ultrasound and establish a method of treating wounds and fighting bacteria without antibiotics when necessary.

Embodiments of the present invention are suitable for use in wound treatment using ultrasonic standing waves through air with no drug. Wound treatment using the systems of the present invention is also possible with a combination of different energy sources, such as an ultrasound, laser, electric current, magnetic field, ultraviolet, microwaves, radio frequency, etc.

## BRIEF DESCRIPTION OF DRAWINGS

To enable a better understanding of the present invention, and to show how the same may be carried into effect, reference will now be made, by way of example, only, to the accompanying drawings, in which:-

Fig.1 is a perspective view of an ultrasound wound treatment system with standing waves, according to the present invention;
Fig. 2 is a schematic, lateral cross-sectional view of another transducer useful with the system of Fig. 1;
Fig. 3 is a schematic, lateral cross-sectional view of a transducer having a bushing;
Fig. 4 is a schematic, lateral cross-sectional view of a transducer of Fig. 3 where the distal tip has been modified to provide a focussed beam; and
Fig. 5 is a schematic, lateral cross-sectional view of an embodiment of the invention in use in a method intended to facilitate dissolution of blood clots.

## DETAILED DESCRIPTION

The following describes a method and device for using ultrasonic standing waves to treat wounds. Ultrasound standing waves occur as a result of incident and reflected waves from a reflective surface that are traveling in opposite directions. The resultant superposition of the two waves forms standing waves, which create ultrasonic radiation pressure. The standing waves, actually ultrasound radiation pressure, occur when the distance between (a) the distal end of a transducer (as a radiant of ultrasound waves) and (b) the reflected surface (e. g., a wound surface) is: n X λ/2, where λ is the wave length and n is a positive integer. The standing waves are more effective in limited space or area as a tube.

The following describes a method and system, which use ultrasound standing wave energy to treat wounds, now with reference to the accompanying drawings. The system comprises a generator of electrical signals and a handpiece having an ultrasound transducer and tip.

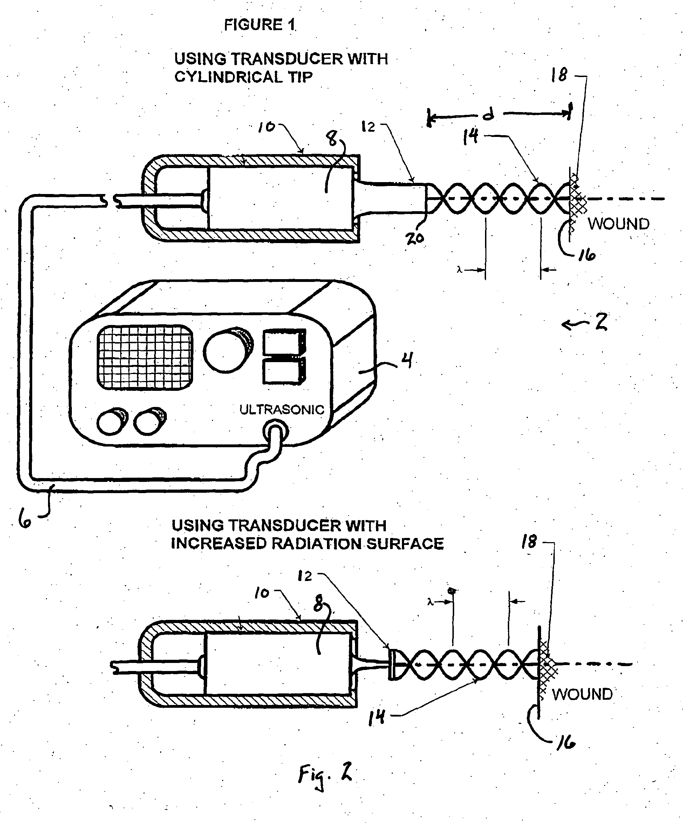

A system for wound treatment according to present invention is illustrated in Fig. 1. The system 2 comprises a signal generator 4 operatively or electrically connected through cable 6 to a transducer 8 in a housing 10. Transducer 8 has an ultrasound tip 12 that generates standing ultrasound waves 14. Standing ultrasound waves 14 are directed to the surface 16 of a wound 18.

Standing ultrasound waves 14 occur when activated ultrasound tip 12 is directed through the air to a wound surface 16 as a result of incident and reflected waves from wound surface 16, which creates ultrasonic radiation pressure. The distal end 20 of transducer tip 12 (as a radiant of ultrasound waves) is preferably a distance d from wound surface 16 (a source of reflected waves). Distance d is related to the wavelength λ of the ultrasound wave or signal by the formula

$$d = n \, X \, \lambda/2$$

where n is a positive integer. To reach this preferred distance and therefore effect wound treatment practice, ultrasound transducer 8 or tip 12 must frequently be moved back and forward toward wound surface 16 by an operator.

The waveform of the ultrasound waves generated by transducer 8 preferably corresponds to the waveform of the electrical signals generated by signal generator 4. For example, electrical signals from signal generator 4 with rectangular, sinusoidal, trapezoidal, or triangular waveforms will cause transducer 8 to produce respective similarly shaped ultrasound waveforms.

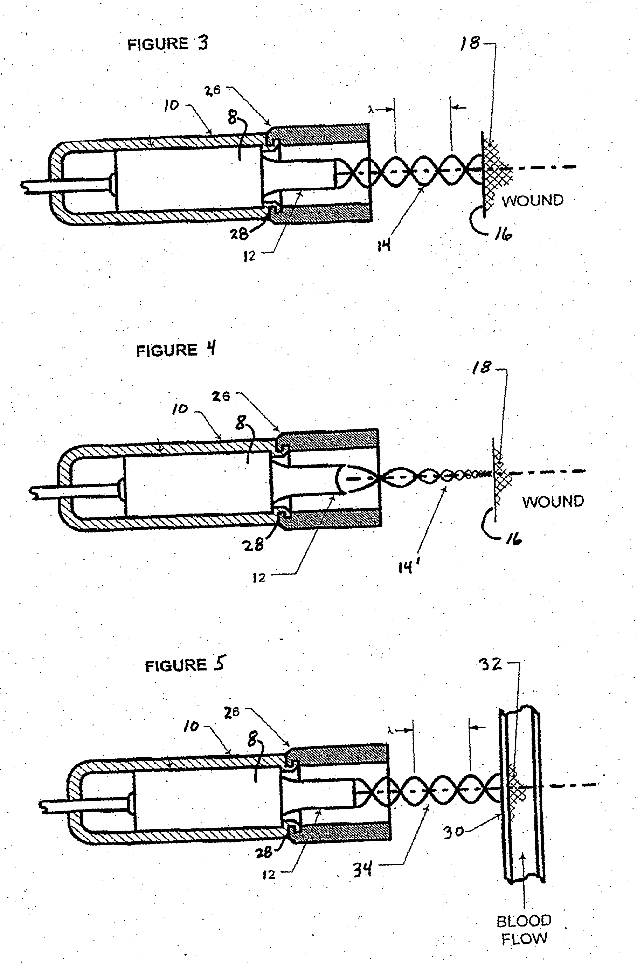

The standing waves are more effective in limited space or area such as a tube. In each of Figs. 3 to 5 a bushing 26 increases ultrasound radiation pressure. Bushing 26 may or may not be disposable part on the distal end 28 of housing 10.

In the embodiment of the invention set forth in Fig. 4 the distal end 20 (not labelled in Fig. 4) of ultrasonic tip 12 has been modified to a concave shape to focus ultrasound waves 14.

One of the possible applications of the method of present invention is the facilitation of dissolution of blood clots by using ultrasound energy. In Fig. 5 ultrasound tip 12 is directed to a blood vessel 30 with clot or clots 32. In this case ultrasound standing waves 34 create cavitation inside blood vessel 30 and dissolve clot or clots 32.

In another embodiment of the present invention a wound can also be treated with a gel or drug. After the gel or drug is applied to the wound surface, ultrasound stranding waves would be directed to the wound. The drug would be activated and penetrate into tissue under ultrasound radiation pressure.

Additional possible application of method using ultrasound standing waves is for the diffusion of grafts to a wound with radiation pressure gently.

The preceding specific embodiments are illustrative of the practice of the invention. It is to be understood, however, that other expedients known to those skilled in the art or disclosed herein may be employed without departing from the scope of the appended claims.

## Claims

1. A system (2) for treating a wound with, ultrasound standing waves (14), comprising
   a generator (4) for generating ultrasound waves,
   an ultrasound transducer (8) operatively connected to said generator and having a distal radiation surface (20), and
   means for adjusting the distance d between the distal radiation surface and a surface of a wound (16), when the distal radiation surface is active and directed to the surface of the wound, so as to create ultrasound standing waves through air from the distal radiation surface to the surface of the wound when the distance d is determined by the formula

$$d = n \times \lambda/2,$$

   wherein $\lambda$ is the wavelength of the ultrasound standing wave and n is a positive integer.

2. The system of Claim 1, wherein the ultrasound transducer operates at a frequency of from 10kHz to $10^3$ MHz.

3. The system of claim 1 or 2, wherein the distal end (28) of a housing (10) of the ultrasound transducer comprises a bushing (26).

4. The system of Claims 1 to 3, wherein, in use of the generator, the ultrasound frequency is modulated.

5. The system of claims 1 to 4, wherein, in use of the generator, the ultrasound frequency is pulsed.

6. The system of Claims 1 to 5, wherein, in use of the generator, the waveform of the ultrasound wave is sinusoidal.

7. The system of Claims 1 to 5, wherein, in use of the generator, the wave form of the ultrasound wave is rectangular.

8. The system of Claims 1 to 5, wherein, in use of the generator, the wave form of the ultrasound wave is trapezoidal.

9. The system of Claims 1 to 5, wherein, in use of the generator, the waveform of the ultrasound wave is triangular.

10. The system of any preceding claim, wherein distal radiation surface (20) is a concave shape such, that, when active, the ultrasound beam thereby directed is focussed.

## Patentansprüche

1. System (2) zum Behandeln einer Wunde mit stehenden Ultraschallwellen (14), umfassend
   einen Generator (4) zum Erzeugen von Ultraschallwellen,
   einen Ultraschallgeber (8), der mit dem Generator wirkverbunden ist und eine distale Strahlungsoberfläche (20) aufweist, und
   Mittel zum Einstellen des Abstands d zwischen der distalen Strahlungsoberfläche und einer Oberfläche einer Wunde (16), wenn die distale Strahlungsoberfläche aktiv ist und auf die Oberfläche der Wunde gerichtet ist, um stehende Ultraschallwellen durch Luft von der distalen Strahlungsoberfläche zu der Oberfläche der Wunde zu erzeugen, wenn der Abstand d durch die Formel

$$d = n \times \lambda/2$$

   gegeben ist,
   wobei $\lambda$ die Wellenlänge der stehenden Ultraschallwelle und n eine positive ganze Zahl ist.

2. System nach Anspruch 1, wobei der Ultraschallgeber bei einer Frequenz von 10kHz bis $10^3$ MHz arbeitet.

3. System nach Anspruch 1 oder 2, wobei das distale Ende (28) eines Gehäuses (10) des Ultraschallgebers eine Hülse (26) umfasst.

**4.** System nach Ansprüchen 1 bis 3, wobei die Ultraschallfrequenz während der Verwendung des Generators moduliert wird.

**5.** System nach Ansprüchen 1 bis 4, wobei die Ultraschallfrequenz während der Verwendung des Generators gepulst wird.

**6.** System nach Ansprüchen 1 bis 5, wobei die Wellenform der Ultraschallwelle während der Verwendung des Generators sinusförmig ist.

**7.** System nach Ansprüchen 1 bis 5, wobei die Wellenform der Ultraschallwelle während der Verwendung des Generators rechteckig ist.

**8.** System nach Ansprüchen 1 bis 5, wobei die Wellenform der Ultraschallwelle während der Verwendung des Generators trapezförmig ist.

**9.** System nach Ansprüchen 1 bis 5, wobei die Wellenform der Ultraschallwelle während der Verwendung des Generators dreieckig ist.

**10.** System nach einem vorstehenden Anspruch, wobei die distale Strahlungsoberfläche (20) eine konkave Form hat, derart, dass der Ultraschallstrahl, der **dadurch** gerichtet wird, fokussiert ist, wenn sie aktiv ist.

**Revendications**

**1.** Système (2) pour traiter une plaie à l'aide d'ondes stationnaires ultrasonores (14), comprenant
un générateur (4) pour générer des ondes ultrasonores,
un transducteur ultrasonore (8) connecté de manière fonctionnelle audit générateur et ayant une surface de radiation distale (20), et
un moyen pour ajuster la distance d entre la surface de radiation distale et une surface d'une plaie (16), lorsque la surface de radiation distale est active et dirigée vers la surface de la plaie, de sorte à créer des ondes stationnaires ultrasonores à travers l'air depuis la surface de radiation distale vers la surface de la plaie lorsque la distance d est déterminée par la formule

$$d = n \times \lambda/2,$$

où $\lambda$ est la longueur d'onde de l'onde stationnaire ultrasonore et n est un entier positif.

**2.** Système de la revendication 1, dans lequel le transducteur ultrasonore fonctionne à une fréquence allant de 10 kHz à $10^3$ MHz.

**3.** Système de la revendication 1 ou 2, dans lequel l'extrémité distale (28) d'un boîtier (10) du transducteur ultrasonore comprend une douille (26).

**4.** Système des revendications 1 à 3, dans lequel, lors de l'utilisation du générateur, la fréquence ultrasonore est modulée.

**5.** Système des revendications 1 à 4, dans lequel, lors de l'utilisation du générateur, la fréquence ultrasonore est pulsée.

**6.** Système des revendications 1 à 5, dans lequel, lors de l'utilisation du générateur, la forme d'onde de l'onde ultrasonore est sinusoïdale.

**7.** Système des revendications 1 à 5, dans lequel, lors de l'utilisation du générateur, la forme d'onde de l'onde ultrasonore est rectangulaire.

**8.** Système des revendications 1 à 5, dans lequel, lors de l'utilisation du générateur, la forme d'onde de l'onde ultrasonore est trapézoïdale.

**9.** Système des revendications 1 à 5, dans lequel, lors de l'utilisation du générateur, la forme d'onde de l'onde ultrasonore est triangulaire.

**10.** Système de l'une des revendications précédentes, dans lequel la surface de radiation distale (20) a une forme concave de telle sorte que, lorsqu'elle est active, le faisceau ultrasonore ainsi dirigé est focalisé.

## FIGURE 1

**USING TRANSDUCER WITH
CYLINDRICAL TIP**

WOUND

**USING TRANSDUCER WITH
INCREASED RADIATION SURFACE**

WOUND

Fig. 2

FIGURE 3

FIGURE 4

FIGURE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3765606 A
- US 4659014 A
- US 5104042 A
- US 4930700 A
- US 4153201 A
- US 4655393 A
- US 5516043 A
- US 5835678 A
- US 5879364 A
- US 5843139 A
- US 4294407 A

- US 5347998 A
- US 5520166 A
- US 5960792 A
- US 6095141 A
- US 6102298 A
- US 6098620 A
- US 6026808 A
- US 6106547 A
- US 6036661 A
- US 5664570 A